Europäisches Patentamt

European Patent Office

Office européen des brevets

(19)

(11) **EP 1 069 948 B1**

(12) **EUROPÄISCHE PATENTSCHRIFT**

(45) Veröffentlichungstag und Bekanntmachung des Hinweises auf die Patenterteilung:
**23.06.2004 Patentblatt 2004/26**

(21) Anmeldenummer: **99917878.3**

(22) Anmeldetag: **26.03.1999**

(51) Int Cl.$^7$: **B01J 23/887**, C07C 51/25

(86) Internationale Anmeldenummer:
**PCT/EP1999/002081**

(87) Internationale Veröffentlichungsnummer:
**WO 1999/051341 (14.10.1999 Gazette 1999/41)**

(54) **MULTIMETALLOXIDMASSEN MIT EINER ZWEI-PHASIGEN STRUKTUR**

MULTI-METAL OXIDE MATERIALS WITH A TWO-PHASE STRUCTURE

MATIERES CONSTITUEES D'OXYDES DE PLUSIEURS METAUX, PRESENTANT UNE STRUCTURE A DEUX PHASES

(84) Benannte Vertragsstaaten:
**BE DE FR NL**

(30) Priorität: **06.04.1998 DE 19815278**

(43) Veröffentlichungstag der Anmeldung:
**24.01.2001 Patentblatt 2001/04**

(73) Patentinhaber: **BASF AKTIENGESELLSCHAFT**
**67056 Ludwigshafen (DE)**

(72) Erfinder:
 • **HIBST, Hartmut**
  **D-69198 Schriesheim (DE)**
 • **UNVERRICHT, Signe**
  **D-68169 Mannheim (DE)**

(56) Entgegenhaltungen:
 **EP-A- 0 235 760      EP-A- 0 668 104**
 **EP-A- 0 686 600      EP-A- 0 758 562**
 **DE-A- 2 407 677      DE-A- 2 635 031**
 **DE-A- 4 302 991      DE-A- 4 405 060**

**Beschreibung**

Die vorliegende Erfindung betrifft Multimetalloxidmassen der allgemeinen Formel I

$$(A)_p \, (B)_q \qquad\qquad (I),$$

in der die Variablen folgende Bedeutung haben:

A =    $Mo_{12}V_aX^1_bX^2_cX^3_dX^4_eX^5_fX^6_gO_x$,

B =    $X^7_1Sb_hH_iO_y$,

$X^1$ =    W, Nb, Ta, Cr und/oder Ce, vorzugsweise W, Nb und/oder Cr,

$X^2$ =    Cu, Ni, Co, Fe, Mn und/oder Zn, vorzugsweise Cu, Ni, Co und/oder Fe,

$X^3$ =    Sb und/oder Bi, vorzugsweise Sb,

$X^4$ =    Li, Na, K, Rb, Cs und/oder H, vorzugsweise Na und/oder K,

$X^5$ =    Mg, Ca, Sr und/oder Ba, vorzugsweise Ca, Sr und/oder Ba,

$X^6$ =    Si, Al, Ti und/oder Zr, vorzugsweise Si, Al und/oder Ti,

$X^7$ =    Ni und gegebenenfalls eines oder mehrere der Elemente aus der Gruppe umfassend Cu, Zn, Co, Fe, Cd, Mn, Mg, Ca, Sr und Ba,

a =    1 bis 8, vorzugsweise 2 bis 6,

b =    0,2 bis 5, vorzugsweise 0,5 bis 2,5,

c =    0 bis 23, vorzugsweise 0 bis 4,

d =    0 bis 50, vorzugsweise 0 bis 3,

e =    0 bis 2, vorzugsweise 0 bis 0,3,

f =    0 bis 5, vorzugsweise 0 bis 2,

g =    0 bis 50, vorzugsweise 0 bis 20,

h =    0,1 bis 50, vorzugsweise 0,2 bis 20, und besonders bevorzugt 0,2 bis 5,

i =    0 bis 50, vorzugsweise 0 bis 20, und besonders bevorzugt 0 bis 12,

x,y =    Zahlen, die durch die Wertigkeit und Häufigkeit der von Sauerstoff verschiedenen Elemente in (I) bestimmt werden und

p,q =    von Null verschiedene Zahlen, deren Verhältnis p/q 20 : 1 bis 1 : 80, vorzugsweise 10 : 1 bis 1 : 35 und besonders bevorzugt 2 : 1 bis 1 : 3 beträgt,

die den Anteil $(A)_p$ in Form dreidimensional ausgedehnter Bereiche A der chemischen Zusammensetzung

   A :

$$Mo_{12}V_aX^1{}_bX^2{}_cX^3{}_dX^4{}_eX^5{}_fX^6{}_gO_x$$

und den Anteil $(B)_q$ in Form dreidimensional ausgedehnter Bereiche B der chemischen Zusammensetzung

B :

$$X^7{}_1Sb_hH_iO_y$$

enthalten, wobei die Bereiche A, B relativ zueinander wie in einem Gemisch aus feinteiligem A und feinteiligem B verteilt sind,
mit der Maßgabe, daß zur Herstellung der Multimetalloxidmassen (I) wenigstens ein getrennt vorgebildetes Oxometallat B,

$$X^7{}_1Sb_hH_iO_y,$$

mitverwendet wird, das dadurch erhältlich ist, daß man von geeigneten Quellen der elementaren Konstituenten des Oxometallats B, die wenigstens einen Teil des Antimons in der Oxidationsstufe +5 enthalten, ein Trockengemisch herstellt und dieses bei Temperaturen von 200 bis <600°C, vorzugsweise 200 bis ≤580°C und besonders bevorzugt 250 bis ≤550°C, calciniert.

Außerdem betrifft vorliegende Erfindung Verfahren zur Herstellung von Multimetalloxidmassen (I) sowie deren Verwendung als Katalysatoren für die gasphasenkatalytische Oxidation von Acrolein zu Acrylsäure.

Die WO 96/27437 betrifft Multimetalloxidmassen, die die Elemente Mo, V, Cu und Sb als wesentliche Bestandteile enthalten und deren Röntgenbeugungsdiagramm bei einem 2θ - Wert von 22,2° die intensitätsstärkste Linie aufweist. Die WO 96/27437 empfiehlt diese Multimetalloxidmassen als geeignete Katalysatoren für die gasphasenkatalytische Oxidation von Acrolein zu Acrylsäure. Ferner empfiehlt die WO 96/27437 als Antimonquelle zur Herstellung dieser Multimetalloxidmassen auf $Sb_2O_3$ zurückzugreifen. Eine Vorabherstellung einer Sb - haltigen Komponente lehrt die WO 96/27437 nicht.

Die EP - B 235760 betrifft ein Verfahren zur Herstellung von Sb, Mo, V und/oder Nb enthaltenden Multimetalloxidmassen, die als Katalysatoren für die gasphasenkatalytische Oxidation von Acrolein zu Acrylsäure geeignet sind. Als Antimonquelle zur Herstellung dieser Multimetalloxidmassen empfiehlt die EP - B 235760 ein vorab hergestelltes, bei Temperaturen von 600 bis 900°C calciniertes, Antimonat einzusetzen.

Nachteilig an den Multimetalloxidmassen des Standes der Technik ist, daß bei ihrer Verwendung als Katalysatoren für die gasphasenkatalytische Oxidation von Acrolein zu Acrylsäure ihre Aktivität und die Selektivität der Acrylsäurebildung nicht in vollem Umfang zu befriedigen vermag.

Die Aufgabe der vorliegenden Erfindung bestand daher darin, neue Multimetalloxidmassen zur Verfügung zu stellen, die bei einer Verwendung als Katalysatoren für die gasphasenkatalytische Oxidation von Acrolein zu Acrylsäure die Nachteile der Katalysatoren des Standes der Technik allenfalls noch in einem verringerten Umfang aufweisen.

Demgemäß wurden die eingangs definierten Multimetalloxidmassen (I) gefunden.

Ganz besonders bevorzugte Massen (I) sind solche, deren Bereiche A eine Zusammensetzung der nachfolgenden allgemeinen Formel (II) aufweisen,

$$Mo_{12}V_a,X^1{}_b,X^2{}_c,X^5{}_f,X^6{}_g,O_x, \qquad (II),$$

mit

$X^1 =$      W und/oder Nb,

$X^2 =$      Cu und/oder Ni,

$X^5 =$      Ca und/oder Sr,

$X^6 =$      Si und/oder Al,

a' =      2 bis 6,

b' = 0,5 bis 2,5,

c' = 0 bis 4,

f' = 0 bis 2,

g' = 0 bis 2 und

x' = eine Zahl, die durch die Wertigkeit und Häufigkeit der von Sauerstoff verschiedenen Elemente in (II) bestimmt wird.

Ferner ist es von Vorteil, wenn wenigstens einer der Anteile $(A)_p$, $(B)q$ der erfindungsgemäßen Multimetalloxidmassen (I) in den letzteren in Form dreidimensional ausgedehnter Bereiche der chemischen Zusammensetzung A bzw. B enthalten ist, deren Größtdurchmesser $d_A$ bzw. $d_B$ (längste durch den Schwerpunkt des Bereichs gehende Verbindungsstrecke zweier auf der Oberfläche (Grenzfläche) des Bereichs befindlicher Punkte) > 0 bis 300 $\mu$m, vorzugsweise 0,01 bis 100 $\mu$m, besonders bevorzugt 0,05 bis 50 $\mu$m und ganz besonders bevorzugt 0,05 bis 20 $\mu$m betragen.

Selbstverständlich können die Größtdurchmesser aber auch 0,05 bis 1,0 $\mu$m oder 75 bis 125 $\mu$m betragen (die experimentelle Ermittlung der Größtdurchmesser gestattet z. B. eine Mikrostrukturanalyse mittels eines Rasterelektronenmikroskops (REM)).

In der Regel liegt der Anteil $(B)q$ in den erfindungsgemäßen Multimetalloxidmassen im wesentlichen in kristalliner Form vor. D. h., in der Regel bestehen die Bereiche B im wesentlichen aus kleinen Kristalliten, deren Größtausdehnung in typischer Weise 0,05 bis 20 $\mu$m beträgt. Selbstverständlich kann der Anteil $(B)_q$ aber auch amorph und/ oder kristallin vorliegen.

Insbesondere sind solche erfindungsgemäßen Multimetalloxidmassen bevorzugt, deren Bereiche B im wesentlichen aus Kristalliten bestehen, die den Trirutil-Strukturtyp des $\alpha$- und/oder $\beta$-Kupferantimonats $CuSb_2O_6$ aufweisen. $\alpha$-$CuSb_2O_6$ kristallisiert in einer tetragonalen Trirutil-Struktur (E.-O. Giere et al., J. Solid State Chem. 131 (1997) 263-274), während $\beta$-$CuSb_2O_6$ eine monoklin verzerrte Trirutil-Struktur aufweist (A. Nakua et al., J. Solid State Chem. 91 (1991) 105-112 oder Vergleichsdiffraktogramm in Karteikarte 17-284 in der JCPDS-ICDD-Kartei 1989). Darüber hinaus werden Bereiche B bevorzugt, die die Pyrochlore-Struktur des Minerals Partzite, eines Kupfer-Antimon-Oxid-Hydroxyds mit der variablen Zusammensetzung $Cu_ySb_{2-x}(O, OH, H_2O)_{6-7}$ ($y \leq 2, 0 \leq x \leq 1$) aufweisen (B. Mason et al., Mineral. Mag. 30 (1953) 100-112 oder Vergleichsdiagramm in Karteikarte 7-303 der JCPDS-ICDD-Kartei 1996).

Des weiteren können die Bereiche B aus Kristalliten bestehen, die die Struktur des Kupferantimonats $Cu_9Sb_4O_{19}$ (S. Shimada et al., Chem. Lett. 1983, 1875-1876 oder S. Shimada et al., Thermochim. Acta 133 (1988) 73-77 oder Vergleichsdiagramm in Karteikarte 45-54 der JCPDS-ICDD-Kartei) und/oder die Struktur von $Cu_4SbO_{4,5}$ (S. Shimada et al., Thermochim. Acta 56 (1982) 73-82 oder S. Shimada et al., Thermochim. Acta 133 (1988) 73-77 oder Vergleichsdiagramm in Karteikarte 36-1106 der JCPDS-ICDD-Kartei) aufweisen.

Selbstverständlich können die Bereiche B auch aus Kristalliten bestehen, die ein Gemenge aus den vorgenannten Strukturen darstellen.

Die erfindungsgemäßen Massen (I) sind in einfacher Weise z. B. dadurch erhältlich, daß man zunächst Oxometallate B,

$$X^7{}_1Sb_hH_iO_y,$$

in feinteiliger Form als Ausgangsmasse 1 getrennt vorbildet. Die Oxometallate B können dadurch hergestellt werden, daß man von geeigneten Quellen ihrer elementaren Konstituenten ein vorzugsweise inniges, zweckmäßigerweise feinteiliges, Trockengemisch herstellt und dieses bei Temperaturen von 200 bis 1200°C, vorzugsweise 200 bis 850°C, besonders bevorzugt 250 bis < 600°C, häufig $\leq$ 550°C, calciniert (in der Regel 10 min bis mehrere Stunden). Erfindungswesentlich ist nur, daß wenigstens ein Teil der Oxometallate B der Ausgangsmasse 1 (nachfolgend Oxometallate B* genannt) dadurch erhältlich ist, daß man von geeigneten Quellen der elementaren Konstituenten des Oxometallats B, die wenigstens einen Teil des Antimons in der Oxidationsstufe +5 enthalten, ein vorzugsweise inniges, zweckmäßigerweise feinteiliges. Trockengemisch herstellt und dieses bei Temperaturen von 200 bis < 600°C, vorzugsweise 200 bis $\leq$580°C, besonders bevorzugt 250 bis $\leq$ 550°C, häufig $\leq$ 500°C, calciniert (in der Regel 10 min bis mehrere Stunden). Die Calcination der Vorläufer der Oxometallate B kann generell unter Inertgas, aber auch unter einem Gemisch aus Inertgas und Sauerstoff wie z. B. Luft oder auch unter reinem Sauerstoff erfolgen. Eine Calcination unter einer reduzierenden Atmosphäre ist auch möglich. In der Regel nimmt die erforderliche Calcinationsdauer mit zunehmender Calcinierungstemperatur ab. Mit Vorteil beträgt der Anteil der Oxometallate B* an der feinteiligen Ausgangsmasse 1 wenigstens 10, besser wenigstens 20, häufig wenigstens 30 oder wenigstens 40, bevorzugt wenigstens 50, noch besser wenigstens 60, besonders bevorzugt wenigstens 70 oder wenigstens 80, häufig wenigstens 90 oder 95 und ganz besonders bevorzugt 100 Gew.-%, bezogen auf die Ausgangsmasse 1.

Oxometallate B* sind z. B. nach den in der DE-A 24 076 77 ausführlich beschriebenen Herstellweisen erhältlich. Unter diesen ist diejenige Verfahrensweise bevorzugt, bei der man Antimontrioxid und/oder $Sb_2O_4$ in wäßrigem Medium mittels Wasserstoffperoxid in einer Menge, die der stöchiometrischen gleich ist oder diese übersteigt, bei Temperaturen zwischen 40 und $100°C$ zu Antimon (V)-Oxidhydroxidhydrat oxidiert, bereits vor dieser Oxidation, während dieser Oxidation und/oder nach dieser Oxidation wäßrige Lösungen und/oder Suspensionen von geeigneten Ausgangsverbindungen der übrigen elementaren Konstituenten des Oxometallats B* zusetzt, anschließend das resultierende wäßrige Gemisch trocknet (vorzugsweise sprühgetrocknet (Eingangstemperatur : 250 bis $600°C$, Ausgangstemperatur: 80 bis $130°C$)) und danach das innige Trockengemisch wie beschrieben calciniert.

Bei dem wie eben beschriebenen Verfahren kann man z. B. wäßrige Wasserstoffperoxidlösungen mit einem $H_2O_2$ - Gehalt von 5 bis 33 oder mehr Gew.-% verwenden. Eine nachträgliche Zugabe von geeigneten Ausgangsverbindungen der übrigen elementaren Konstituenten des Oxometallats B* ist vor allem dann zu empfehlen, wenn diese das wasserstoffperoxid katalytisch zu zersetzen vermögen. Selbstverständlich könnte man aber auch das entstandene Antimon (V)-Oxidhydroxidhydrat aus dem wäßrigen Medium isolieren und z. B. trocken mit geeigneten feinteiligen Ausgangsverbindungen der übrigen elementaren Konstituenten des Oxometallats B* innig vermischen und anschließend dieses innige Gemisch wie beschrieben calcinieren.

Wesentlich ist, daß es sich bei den Elementquellen der Oxometallate B, B* entweder bereits um Oxide handelt, oder um solche Verbindungen, die durch Erhitzen, gegebenenfalls in Anwesenheit von Sauerstoff, in Oxide überführbar sind.

Neben den Oxiden kommen daher als Ausgangsverbindungen vor allem Halogenide, Nitrate, Formiate, Oxalate, Acetate, Carbonate und/oder Hydroxide in Betracht (Verbindungen wie $NH_4OH$, $NH_4CHO_2$, $CH_3COOH$, $NH_4CH_3CO_2$ oder Ammoniumoxalat, die spätestens beim Calcinieren zu vollständig gasförmig entweichenden Verbindungen zerfallen und/oder zersetzt werden können, können zusätzlich eingearbeitet werden). Generell kann zur Herstellung von Oxometallaten B das innige Vermischen der Ausgangsverbindungen in trockener oder in nasser Form erfolgen. Erfolgt es in trockener Form, werden die Ausgangsverbindungen zweckmäßigerweise als feinteilige Pulver eingesetzt. Vorzugsweise erfolgt das innige Vermischen jedoch in nasser Form. Üblicherweise werden die Ausgangsverbindungen dabei in Form einer wäßrigen Lösung ünd/oder Suspension miteinander vermischt. Nach Abschluß des Mischvorgangs wird die fluide Masse getrocknet und nach Trocknung calciniert. Vorzugsweise erfolgt die Trocknung durch Sprühtrocknung.

Nach erfolgter Calcinierung können die Oxometallate B, B* nochmals zerkleinert (z.B. durch Naß- oder Trockenmahlen, z.B. in der Kugelmühle oder durch Strahlmahlen) und aus dem dabei erhältlichen, häufig aus im wesentlichen kugelförmigen Partikeln bestehenden, Pulver die Kornklasse mit einem im für das erfindungsgemäße Multimetalloxid (I) gewünschten Größtdurchmesserbereich liegenden Korngrößtdurchmesser (in der Regel > 0 bis 300 μm, üblicherweise 0,01 bis 200 μm, vorzugsweise 0,01 bis 100 μm und ganz besonders bevorzugt 0,05 bis 20 μm) durch in an sich bekannter Weise durchzuführendes Klassieren (z.B. Naß- oder Trockensiebung) abgetrennt werden.

Eine bevorzugte Herstellweise von Oxometallaten B* der allgemeinen Formel $(X^7_1)Sb_hH_iO_y$ mit $X^7 = $ Ni und gegebenenfalls Cu und/oder Zn, besteht darin, Antimontrioxid und/oder $Sb_2O_4$ in wäßrigem Medium mittels Wasserstoffperoxid zunächst in eine, vorzugsweise feinteilige, Sb(V)-Verbindung, z.B. Sb(V)-Oxidhydroxidhydrat, überzuführen, die dabei resultierende wäßrige Suspension mit einer ammoniakalischen wäßrigen Lösung von Ni-Carbonat und gegebenenfalls Zn- und/oder Cu-Carbonat (das z.B. die Zusammensetzung $Cu_2(OH)_2CO_3$ aufweisen kann) zu versetzen, die resultierende wäßrige Mischung zu trocknen, z.B. wie beschrieben sprühzutrocknen, und das erhaltene Pulver, gegebenenfalls nach anschließendem Verkneten mit Wasser und darauffolgendem Verstrangen und Trocknen, wie beschrieben zu calcinieren.

Im Fall von von Oxometallaten B* verschiedenen Oxometallaten B erweist es sich als besonders günstig, von einer wäßrigen Antimontrioxidsuspension auszugehen und darin die $X^7$ - Elemente als Nitrat und/oder Acetat zu lösen, die resultierende wäßrige Mischung wie beschrieben sprühzutrocknen und dann das resultierende Pulver wie beschrieben zu calcinieren.

Der Anteil des Ni an $X^7$ kann erfindungsgemäß $\geq$ 1 mol-%, oder $\geq$ 5 mol-%, oder $\geq$ 10 mol-% oder $\geq$ 20 mol-%, aber auch $\geq$ 30 mol-%, oder $\geq$ 40 mol-%, oder $\geq$ 50 mol-% betragen. Selbstverständlich kann der vorgenannte Anteil des Ni aber auch bei $\geq$ 60 mol-%, oder $\geq$ 70 mol-%, oder $\geq$ 80 mol-% oder $\geq$ 90 mol-% liegen. Ni kann aber auch alleine $X^7$ darstellen.

Die wie beschrieben vorgebildeten Ausgangsmassen 1 können dann zur Herstellung von Multimetalloxidmassen (I) mit geeigneten Quellen der elementaren Konstituenten der Multimetalloxidmasse A,

$$Mo_{12}V_aX^1_bX^2_cX^3_dX^4_eX^5_fX^6_gO_x,$$

im gewünschten Mengenverhältnis in innigen Kontakt gebracht und ein daraus resultierendes Trockengemisch bei einer Temperatur von 250 bis 500°C calciniert werden, wobei die Calcination unter Inertgas (z.B. $N_2$), einem Gemisch aus Inertgas und Sauerstoff (z.B. Luft), reduzierend wirkenden Gasen wie Kohlenwasserstoffen (z.B. Methan), Aldehyden (z.B. Acrolein) oder Ammoniak aber auch unter einem Gemisch aus $O_2$ und reduzierend wirkenden Gasen (z. B. allen vorgenannten) erfolgen kann, wie es beispielsweise in der DE - A 43 359 73 beschrieben wird. Bei einer Calcination unter reduzierenden Bedingungen ist zu beachten, daß die metallischen Konstituenten nicht bis zum Element reduziert werden. Die Calcinationsdauer erstreckt sich in der Regel über einige Stunden und nimmt üblicherweise mit zunehmender Calcinierungstemperatur ab. Wesentlich für die Quellen der elementaren Konstituenten der Multimetalloxidmasse A ist dabei, wie allgemein bekannt, nur, daß es sich entweder bereits um Oxide handelt oder um solche Verbindungen, die durch Erhitzen, wenigstens in Anwesenheit von Sauerstoff, in Oxide überführbar sind. Neben den Oxiden kommen als Ausgangsverbindungen vor allem Halogenide, Nitrate, Formiate, Oxalate, Citrate, Acetate, Carbonate oder Hydroxide in Betracht. Geeignete Ausgangsverbindungen des Mo, V, W und Nb sind auch deren Oxoverbindungen (Molybdate, Vanadate, Wolframate und Niobate) bzw. die von diesen abgeleiteten Säuren.

Das innige Inkontaktbringen der Ausgangsmasse 1 mit den Quellen der Multimetalloxidmasse A (Ausgangsmasse 2) kann sowohl trocken als auch naß erfolgen. Im letzteren Fall muß lediglich darauf geachtet werden, daß die vorgebildeten Multimetalloxide B, B* nicht in Lösung gehen. In wäßrigem Medium ist letzteres bei pH - Werten, die nicht zu stark von 7 abweichen und bei Temperaturen $\leq 60°C$ bzw. $\leq 40°C$, üblicherweise gewährleistet. Erfolgt das innige Inkontaktbringen naß, wird anschließend normalerweise zu einer Trockenmasse getrocknet (vorzugsweise durch Sprühtrocknen).

Eine solche Trockenmasse fällt im Rahmen eines trockenen Mischens automatisch an.

Als mögliche Mischungsvarianten kommen damit z. B. in Betracht:

a. eine trockene, feinteilige, vorgebildete Ausgangsmasse 1 mit trockenen, feinteiligen Ausgangsverbindungen der elementaren Konstituenten des gewünschten Multimetalloxids A im gewünschten Mengenverhältnis in einem Mischer, Kneter oder in einer Mühle mischen;

b. ein feinteiliges Multimetalloxid A vorbilden durch inniges Mischen geeigneter Ausgangsverbindungen seiner elementaren Konstituenten (trocken oder naß) und anschließendes Calcinieren der daraus resultierenden innigen Trockenmischung bei Temperaturen von 250 bis 500°C (bezüglich Calcinationsdauer, Calcinationsatmosphäre und Elementquellen gilt das oben gesagte); das vorgebildete Multimetalloxid A feinteilig gestalten und mit der feinteiligen Ausgangsmasse 1 im gewünschten Mengenverhältnis wie in a. mischen; bei dieser Mischungsvariante ist ein abschließendes Calcinieren der resultierenden Mischung nicht essentiell;

c. in eine wäßrige Lösung und/oder Suspension von Ausgangsverbindungen der elementaren Konstituenten des gewünschten Multimetalloxids A die erforderliche Menge vorgebildeter Ausgangsmasse 1 einrühren und anschließend sprühtrocknen; selbstverständlich kann anstelle der Ausgangsverbindungen der elementaren Konstituenten des gewünschten Multimetalloxids A auch ein bereits gemäß b. vorgebildetes Multimetalloxid A selbst eingesetzt werden.

Natürlich können auch alle zwischen a., b. und/oder c. liegenden Mischungsvarianten angewendet werden. Das resultierende innige Trockengemisch kann anschließend wie beschrieben calciniert und danach zur gewünschten Katalysatorgeometrie geformt werden oder umgekehrt. Prinzipiell kann das calcinierte (oder bei Anwendung von Mischungsvariante b. gegebenenfalls uncalcinierte) Trockengemisch aber auch als Pulverkatalysator eingesetzt werden.

Eigene Untersuchungen haben ergeben, daß beim Calcinieren des die Ausgangsmasse 1 und die Ausgangsmasse 2 umfassenden Trockengemisches im wesentlichen kein Verschmelzen (Ineinanderlösen) der Bestandteile der Ausgangsmasse 1 mit jenen der Ausgangsmasse 2 stattfindet und der Strukturtyp der in der Ausgangsmasse 1 enthaltenen Kristallite als solcher häufig im wesentlichen erhalten bleibt.

Dies eröffnet, wie bereits angedeutet, die Möglichkeit, nach Mahlen des vorgebildeten Ausgangsgemisches 1 aus dem dabei erhältlichen, häufig aus im wesentlichen kugelförmigen Partikeln bestehenden Pulver, die Kornklasse mit dem im für die Multimetalloxidmasse (I) gewünschten Größtdurchmesserbereich liegenden Korngrößtdurchmesser (in der Regel > 0 bis 300 µm, vorzugsweise 0,01 bis 100 µm und besonders bevorzugt 0,05 bis 20 µm) durch in an sich bekannter Weise durchzuführendes Klassieren (z.B. Naß - oder Trockensiebung) abzutrennen und so zur Herstellung der gewünschten Multimetalloxidmasse maßgeschneidert einzusetzen.

Bei Verwendung der erfindungsgemäßen Multimetalloxidmassen (I) als Katalysatoren für die gasphasenkatalytische Oxidation von Acrolein zu Acrylsäure erfolgt die Formgebung zur gewünschten Katalysatorgeometrie vorzugsweise durch Aufbringen auf vorgeformte inerte Katalysatorträger, wobei das Aufbringen vor oder nach der abschließenden Calcination erfolgen kann. Dabei können die üblichen Trägermaterialien wie poröse oder unporöse Aluminiumoxide, Siliciumdioxid, Thoriumdioxid, Zirkondioxid, Siliciumcarbid oder Silicate wie Magnesium- oder Aluminiumsilicat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Unter diesen sind wiederum Kugeln besonders vorteilhaft. Von besonderem Vorteil ist die Verwendung von im wesentlichen unporösen, oberflächenrauhen, kugelförmigen Trägern aus Steatit, deren Durchmesser 1 bis 8 mm, bevorzugt 4 bis 5 mm beträgt. Die Schichtdicke der Aktivmasse wird zweckmäßigerweise als im Bereich 50 bis 500 μm, bevorzugt im Bereich 150 bis 250 μm liegend, gewählt. Es sei an dieser Stelle darauf hingewiesen, daß bei der Herstellung solcher Schalenkatalysatoren zur Beschichtung der Trägerkörper die aufzubringende Pulvermasse in der Regel befeuchtet und nach dem Aufbringen, z.B. mittels heißer Luft, wieder getrocknet wird.

Die Beschichtung der Trägerkörper wird zur Herstellung der Schalenkatalysatoren in der Regel in einem geeigneten drehbaren Behälter ausgeführt, wie er z.B. aus der DE-A 2909671 oder aus der EP-A 293859 vorbekannt ist. In der Regel wird die relevante Masse vor der Trägerbeschichtung calciniert.

In geeigneter Weise kann das Beschichtungs- und Calcinierungsverfahren gemäß der EP-A 293 859 in an sich bekannter Weise so angewendet werden, daß die resultierenden Multimetalloxidaktivmassen eine spezifische Oberfläche von 0,50 bis 150 m²/g, ein spezifisches Porenvolumen von 0,10 bis 0,90 cm³/g und eine solche Porendurchmesser-Verteilung aufweisen, daß auf die Durchmesserbereiche 0,1 bis < 1 μm, 1,0 bis < 10 μm und 10 μm bis 100 μm jeweils wenigstens 10 % des Porengesamtvolumens entfallen. Auch können die in der EP-A 293 859 als bevorzugt genannten Porendurchmesser-Verteilungen eingestellt werden.

Selbstverständlich können die erfindungsgemäßen Multimetalloxidmassen auch als Vollkatalysatoren betrieben werden. Diesbezüglich wird das die Ausgangsmassen 1 und 2 umfassende innige Trockengemisch vorzugsweise unmittelbar zur gewünschten Katalysatorgeometrie verdichtet (z.B. mittels Tablettieren, Extrudieren oder Strangpressen), wobei gegebenenfalls an sich übliche Hilfsmittel, z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können, und calciniert. Generell kann auch hier vor der Formgebung calciniert werden. Bevorzugte Vollkatalysatorgeometrie sind Hohlzylinder mit einem Außendurchmesser und einer Länge von 2 bis 10 mm und einer Wandstärke von 1 bis 3 mm.

Die erfindungsgemäßen Multimetalloxidmassen eignen sich insbesondere als Katalysatoren mit erhöhter Aktivität und Selektivität (bei vorgegebenem Umsatz) für die gasphasenkatalytische Oxidation von Acrolein zu Acrylsäure. Normalerweise wird bei dem Verfahren Acrolein eingesetzt, das durch die katalytische Gasphasenoxidation von Propen erzeugt wurde. In der Regel werden die Acrolein enthaltenden Reaktionsgase dieser Propenoxidation ohne Zwischenreinigung eingesetzt. Üblicherweise wird die gasphasenkatalytische Oxidation des Acroleins in Rohrbündelreaktoren als heterogene Festbettoxidation ausgeführt. Als Oxidationsmittel wird in an sich bekannter Weise Sauerstoff, zweckmäßigerweise mit inerten Gasen verdünnt (z.B. in Form von Luft), eingesetzt. Geeignete Verdünnungsgase sind z.B. $N_2$, $CO_2$, Kohlenwasserstoff, rückgeführte Reaktionsabgase und/oder Wasserdampf. In der Regel wird bei der Acroleinoxidation ein Acrolein:Sauerstoff:Wasserdampf:Inertgas-Volumenverhältnis von 1:(1 bis 3):(0 bis 20):(3 bis 30), vorzugsweise von 1:(1 bis 3):(0,5 bis 10):(7 bis 18) eingestellt. Der Reaktionsdruck beträgt im allgemeinen 1 bis 3 bar und die Gesamtraumbelastung beträgt vorzugsweise 1000 bis 3500 Nl/l/h. Typische Vielrohr-Festbettreaktoren sind z.B. in den Schriften DE-A 2830765, DE-A 2 201 528 oder US-A 3 147 084 beschrieben. Die Reaktionstemperatur wird üblicherweise so gewählt, daß der Acroleinumsatz bei einfachem Durchgang oberhalb von 90 %, vorzugsweise oberhalb von 98 %, liegt. Im Normalfall sind diesbezüglich Reaktionstemperaturen von 230 bis 330°C erforderlich.

Neben der gasphasenkatalytischen Oxidation von Acrolein zu Acrylsäure vermögen die erfindungsgemäßen Verfahrensprodukte aber auch die gasphasenkatalytische Oxidation anderer organischer Verbindungen wie insbesondere anderer, vorzugsweise 3 bis 6 C-Atome aufweisender, Alkane, Alkanole, Alkanale, Alkene und Alkenole (z.B. Propylen, Methacrolein, tert.-Butanol, Methylether des tert.-Butanol, iso-Buten, iso-Butan oder iso-Butyraldehyd) zu olefinisch ungesättigten Aldehyden und/oder Carbonsäuren, sowie den entsprechenden Nitrilen (Ammoxidation, vor allem von Propen zu Acrylnitril und von iso-Buten bzw. tert.-Butanol zu Methacrylnitril) zu katalysieren. Beispielhaft genannt sei die Herstellung von Acrolein, Methacrolein und Methacrylsäure. Sie eignen sich aber auch zur oxidativen Dehydrierung olefinischer Verbindungen.

Umsatz, Selektivität und Verweilzeit sind in dieser Schrift, falls nichts anderes erwähnt wird, wie folgt definiert:

$$\text{Umsatz U an Acrolein (\%)} = \frac{\text{Molzahl umgesetztes Acrolein}}{\text{Molzahl eingesetztes Acrolein}} \times 100;$$

$$\text{Selektivität S der Acrylsäurebildung \%} = \frac{\text{Molzahl Acrolein umgesetzt zu Acrylsäure}}{\text{Molzahl Acrolein insgesamt umgesetzt}} \times 100;$$

$$\text{Verweilzeit (sec)} = \frac{\text{mit Katalysator gefülltes Leervolumen des Reaktors (1)}}{\text{durchgesetzte Synthese gasmenge (Nl/h)}} \times 3600.$$

Beispiele

I. Katalysatorherstellung

Beispiel

a) Herstellung der Ausgangsmasse 1

Unter Rühren wurden 946,0 g $Sb_2O_3$ mit einem Sb - Gehalt von 83,0 Gew.-% in 4 l Wasser suspendiert. Bei Raumtemperatur (25°C) wurden 822,4 g einer 30 gew.-%igen wäßrigen $H_2O_2$ - Lösung hinzugegeben. Dann wurde die Suspension unter weiterem Rühren innerhalb von 1 Stunde auf 100°C aufgeheizt und bei dieser Temperatur 5 Stunden lang unter Rückfluß gehalten. Anschließend wurde in die 100°C heiße wäßrige Suspension innerhalb von 30 Minuten eine Lösung von 536,0 g $Cu(CH_3COO)_2 \cdot H_2O$ mit einem Cu - Gehalt von 32,0 Gew.-% und 74,6 g $Ni(CH_3COO)_2 \cdot 4 H_2O$ mit einem Ni-Gehalt von 23,6 Gew.-% in 4 l Wasser zugegeben, wobei sich die Temperatur des wäßrigen Gesamtgemisches auf 60°C erniedrigte. Bei dieser Temperatur wurden anschließend 407,9 g einer 25 gew.-%igen wäßrigen Ammoniaklösung zugegeben. Danach wurde die wäßrige Suspension 2 Stunden lang bei 80°C nachgerührt und dann auf Raumtemperatur (25°C) abgekühlt. Schließlich wurde die wäßrige Suspension sprühgetrocknet (Eingangstemperatur: 350°C, Ausgangstemperatur: 110°C). Das resultierende Sprühpulver wurde in einem Drehrohrofen (2 l Innenvolumen) unter Durchleiten von 100 Nl/h Luft stufenweise zunächst innerhalb von 1 Stunde auf 150°C, danach innerhalb von 4 Stunden auf 200°C und schließlich innerhalb von 2 Stunden auf 300°C aufgeheizt und bei letzterer Temperatur 1 Stunde lang gehalten.Anschließend wurde das erhaltene Pulver innerhalb von 1,5 Stunden auf 400°C aufgeheizt und bei dieser Temperatur 1 Stunde lang getempert. Das erhaltene Pulver wies eine spezifische BET - Oberfläche (bestimmt nach DIN 66131 durch Gasadsorption ($N_2$) gemäß Brunauer-Emmet-Teller) von 48,0 $m^2$/g und die Elementstöchiometrie $(Cu_{0,9}Ni_{0,1})Sb_{2,15}O_y$ (y $\leq$ 6,375) auf. Das Pulver zeigte die Röntgenbeugungsreflexe des Minerals Partzite und entsprach damit dem Vergleichsspektrum 7 - 0303 der JCPDS-ICDD-Kartei 1996.

b) Herstellung der Ausgangsmasse 2

In 5030 g Wasser wurden bei 95°C nacheinander 682,4 g Ammoniumheptamolybdattetrahydrat (81,5 Gew.-% $MoO_3$), 131,0 g Ammoniummetavanadat (77,3 Gew.-% $V_2O_5$) und 114,6 g Ammoniumparawolframatheptahydrat (89,0 Gew.-% $WO_3$) gelöst. Der wäßrigen Lösung (Ausgangsmasse 2) lag somit nachfolgende Elementstöchiometrie zugrunde:

$$Mo_{3,86}V_{1,11}W_{0,44} \mathrel{\hat{=}} (Mo_{12}V_{3,45}W_{1,37})_{1,32}.$$

c) Herstellung einer Multimetalloxidmasse M und eines Schalenkatalysators SM

Die vorstehend erhaltene klare, orangefarbene Lösung (Ausgangsmasse 2) wurde auf 25°C abgekühlt und 150,0 g Ammoniumacetat zugegeben. Von der Ausgangsmasse 1 wurden 239,0 g in die auf 25°C abgekühlte wäßrige Lösung eingerührt, so daß das molare Verhältnis der vorgenannten stöchiometrischen Einheiten 0,56 (Ausgangsmasse 1) zu 0,32 (Ausgangsmasse 2) betrug. Die resultierende Suspension wurde bei 25°C 1h lang nachgerührt und anschließend das wäßrige Gemisch sprühgetrocknet. Danach wurde das Sprühpulver mit einem Gemisch aus 70 Gew.-% Wasser und 30 Gew.-% Essigsäure (0,35 kg Flüssigkeit / kg Sprühpulver) verknetet (Kneter LUK 2,5 der Fa. Werner und Pfleiderer). Das erhaltene Knetgut wurde 16 h lang in einem luftdurchströmten Umluftofen bei 110°C getrocknet. Das anschließend zerkleinerte Knetgut wurde in einem mit einem Luft- / Stickstoff-Gemisch (15 Nl/h Luft und 200 Nl/h Stickstoff) durchströmten zylindrischen Drehrohrofen (Innendurchmesser: 12,5 cm, beheizte Länge: 50 cm) calciniert. In die beheizte Zone des Drehrohres wurden 700 g Calciniergut eingebracht. Im Rahmen der Calcinierung wurde zunächst innerhalb von 60 min. auf 325°C aufgeheizt und diese Temperatur dann für 4h gehalten. Anschließend wurde innerhalb von 20 min auf 400°C aufgeheizt und diese Temperatur während 1h aufrechterhalten. Die resultierende katalytisch aktive Multimetalloxidmasse wies folgende Bruttostöchiometrie auf:

$$Mo_{3,86}V_{1,11}W_{0,44}Cu_{0,50}Ni_{0,056}Sb_{1,20}O_x \equiv (Mo_{12}V_{3,45}W_{1,37})_{0,32}\ ((Cu_{0,9}Ni_{0,1})Sb_{2,15}O_y)_{0,56}.$$

Nach dem Mahlen der calcinierten Aktivmasse wurden mit dieser in einer Drehtrommel unporöse und oberflächenrauhe Steatit - Kugeln eines Durchmessers von 4 bis 5 mm in einer Menge von 60 g Aktivpulver je 400 g Steatitkugeln bei gleichzeitigem Zusatz von Wasser beschichtet (Beschichtungsverfahren gemäß DE-A 4 442 346). Anschließend wurde der erhaltene Schalenkatalysator SM mit 110°C heißer Luft getrocknet.

Vergleichsbeispiel

Die Herstellung einer Vergleichsmultimetalloxidmasse VM und eines Vergleichsschalenkatalysators VSM wurde wie im Beispiel vorgenommen, mit dem Unterschied, daß zur Herstellung der Ausgangsmasse 1 beim Calcinieren abschließend nicht 1 h bei 400°C, sondern 1 h bei 800°C getempert wurde.

II. Verwendung der Schalenkatalysatoren aus I. als Katalysatoren für die Gasphasenoxidation von Acrolein zu Acrylsäure

Die Schalenkatalysatoren wurden in einen Rohrreaktor gefüllt (V2A - Stahl, 25 mm Innendurchmesser, 2000 g Katalysatorschüttung, Salzbadtemperierung) und unter Anwendung einer Verweilzeit von 2,0 sec mit einem gasförmigen Gemisch der Zusammensetzung

5 Vol.-% Acrolein,
7 Vol.-% Sauerstoff,
10 Vol.-% Wasserdampf und
78 Vol.-% Stickstoff

beschickt. Die Salzbadtemperatur wurde stets so eingestellt, daß nach beendeter Formierung, bei einfachem Durchgang ein einheitlicher Acroleinumsatz U von 99% resultierte. Das aus dem Reaktor strömende Produktgasgemisch wurde gaschromatographisch analysiert. Die Ergebnisse für die Selektivität der Acrylsäurebildung in Anwendung der verschiedenen Katalysatoren sowie die erforderlichen Salzbadtemperaturen zeigt die nachfolgende Tabelle:

| Katalysator | Salzbadtemperatur (°C) | S% |
| --- | --- | --- |
| SM | 266 | 95,5 |
| VSM | 276 | 93,2 |

**Patentansprüche**

1.    Multimetalloxidmassen der allgemeinen Formel I

$$(A)_p \, (B)_q \qquad\qquad (I),$$

in der die Variablen folgende Bedeutung haben:

A    $= Mo_{12}V_aX^1{}_bX^2{}_cX^3{}_dX^4{}_eX^5{}_fX^6{}_gO_x,$

B    $= X^7{}_1Sb_hH_iO_y,$

$X^1$    $= W, Nb, Ta, Cr$ und/oder $Ce,$

$X^2$    $= Cu, Ni, Co, Fe, Mn$ und/oder $Zn,$

$X^3$    $= Sb$ und/oder $Bi,$

$X^4$    $= Li, Na, K, Rb, Cs$ und/oder $H,$

$X^5$    $= Mg, Ca, Sr$ und/oder $Ba,$

$X^6 =$    $Si, Al, Ti$ und/oder $Zr,$

$X^7 =$    $Ni$ und gegebenenfalls eines oder mehrere der Elemente aus der Gruppe umfassend $Cu, Zn, Co, Fe, Cd,$

Mn, Mg, Ca, Sr und Ba,

a = 1 bis 8,

b = 0,2 bis 5,

c = 0 bis 23,

d = 0 bis 50,

e = 0 bis 2,

f = 0 bis 5,

g = 0 bis 50,

h = 0,1 bis 50,

i = 0 bis 50,

x,y = Zahlen, die durch die Wertigkeit und Häufigkeit der von Sauerstoff verschiedenen Elemente in (I) bestimmt werden und

p,q = von Null verschiedene Zahlen, deren Verhältnis p/q 20 : 1 bis 1 : 80 beträgt,

die den Anteil $(A)_p$ in Form dreidimensional ausgedehnter Bereiche A der chemischen Zusammensetzung.

A :

$$Mo_{12}V_a X^1{}_b X^2{}_c X^3{}_d X^4{}_e X^5{}_f X^6{}_g O_x$$

und den Anteil $(B)_q$ in Form dreidimensional ausgedehnter Bereiche B der chemischen Zusammensetzung

B :

$$X^7{}_1 Sb_h H_i O_y$$

enthalten, wobei die Bereiche A, B relativ zueinander wie in einem Gemisch aus feinteiligem A und feinteiligem B verteilt sind,
mit der Maßgabe, daß zur Herstellung der Multimetalloxidmassen (I) wenigstens ein getrennt vorgebildetes Oxometallat B,

$$X^7{}_1 Sb_h H_i O_y,$$

mitverwendet wird, das dadurch erhältlich ist, daß man von geeigneten Quellen der elementaren Konstituenten des Oxometallats B, die wenigstens einen Teil des Antimons in der Oxidationsstufe +5 enthalten, ein Trockengemisch herstellt und dieses bei Temperaturen von 200 bis < 600°C calciniert.

2. Verfahren zur Herstellung von Multimetalloxiden gemäß Anspruch 1, bei dem man ein Oxometallat B,

$$X^7{}_1 Sb_h H_i O_y,$$

in feinteiliger Form vorbildet und anschließend mit Quellen der elementaren Konstituenten der Multimetalloxidmasse A,

$$Mo_{12}V_aX^1_bX^2_cX^3_dX^4_eX^5_fX^6_gO_x,$$

im gewünschten Mengenverhältnis zu einem Trockengemisch verarbeitet und dieses bei einer Temperatur von 250 bis 500°C calciniert, **dadurch gekennzeichnet, daß** wenigstens eine Teilmenge des Oxometallats B dadurch erhältlich ist, daß man von Quellen der elementaren Konstituenten des Oxometallats B, die wenigstens einen Teil des Antimons in der Oxidationsstufe +5 enthalten, ein Trockengemisch herstellt und dieses bei Temperaturen von 200 bis < 600°C calciniert.

**3.** Verfahren der gasphasenkatalytisch oxidativen Herstellung von Acrylsäure aus Acrolein, **dadurch gekennzeichnet, daß** als Katalysator ein Multimetalloxid gemäß Anspruch 1 verwendet wird.

**Claims**

**1.** A multimetal oxide material of the formula I

$$(A)_p (B)_q \qquad\qquad (I),$$

where

A is $\qquad Mo_{12}V_aX^1_bX^2_cX^3_dX^4_eX^5_fX^6_gO_x,$

B is $\qquad X^7_1Sb_hH_iO_y,$

$X^1$ is $\qquad$ W, Nb, Ta, Cr and/or Ce,

$X^2$ is $\qquad$ Cu, Ni, Co, Fe, Mn and/or zn,

$X^3$ is $\qquad$ Sb and/or Bi,

$X^4$ is $\qquad$ Li, Na, K, Rb, Cs and/or H,

$X^5$ is $\qquad$ Mg, Ca, Sr and/or Ba,

$X^6$ is $\qquad$ Si, Al, Ti and/or Zr,

$X^7$ is $\qquad$ Ni and, if required, one or more of the elements selected from the group consisting of Cu, Zn, Co, Fe, Cd, Mn, Mg, Ca, Sr and Ba,

a is $\qquad$ 1 to 8,

b is $\qquad$ 0.2 to 5,

c is $\qquad$ 0 to 23,

d is $\qquad$ 0 to 50,

e is $\qquad$ 0 to 2,

f is $\qquad$ 0 to 5,

g is $\qquad$ 0 to 50,

h is $\qquad$ 0.1 to 50,

i is        0 to 50,

x and y    are each numbers which are determined by the valency and frequency of the elements in (I) other than oxygen and

p and q    are each numbers which differ from zero and whose ratio p/q is from 20 : 1 to 1 : 80,

which contains the moiety $(A)_p$ in the form of three-dimensional regions A having the chemical composition

A :

$$Mo_{12}V_aX^1{}_bX^2{}_cX^3{}_dX^4{}_eX^5{}_f X^6{}_gO_x$$

and the moiety $(B)_q$ in the form of three-dimensional regions B having the chemical composition
B :

$$X^7{}_1Sb_hH_iO_y,$$

the regions A, B being distributed relative to one another as in a mixture of finely divided A and finely divided B, with the proviso that, for the preparation of the multimetal oxide materials (I), at least one separately preformed oxometallate B,

$$X^7{}_1Sb_hH_iO_y,$$

is present, which is obtainable by preparing a dry blend from suitable sources of the elemental constituents of the oxometallate B which contain at least a part of the antimony in oxidation state +5 and calcining said dry blend at from 200 to < 600°C.

2.  A process for the preparation of a multimetal oxide as claimed in claim 1, in which an oxometallate B,

$$X^7{}_1Sb_hH_iO_y,$$

is preformed in finely divided form and then processed with sources of the elemental constituents of the multimetal oxide material A,

$$Mo_{12}V_aX^1{}_bX^2{}_cX^3{}_dX^4{}_eX^5{}_fX^6{}_gO_x,$$

in the desired ratio to give a dry blend and the latter is calcined at from 250 to 500°C, wherein at least a portion of the oxometallate B is obtainable by preparing a dry blend from sources of the elemental constituents of the oxometallate B which contain at least a part of the antimony in oxidation state +5 and calcining said dry blend at from 200 to < 600°C.

3.  A process for the gas-phase catalytic oxidative preparation of acrylic acid from acrolein, wherein the catalyst used is a multimetal oxide as claimed in claim 1.

**Revendications**

1.  Matières d'oxydes multimétalliques de formule générale I

$$(A)_p (B)_q \qquad\qquad (I),$$

dans laquelle les variables ont la signification suivante :

A        représente $Mo_{12}V_aX^1_bX^2_cX^3_dX^4_eX^5_fX^6_gO_x$,
B        représente $X^7_1Sb_hH_iO_y$,
$X^1$       représente W, Nb, Ta, Cr et/ou Ce,
$X^2$       représente Cu, Ni, Co, Fe, Mn et/ou Zn,
$X^3$       représente Sb et/ou Bi,
$X^4$       représente Li, Na, K, Rb, Cs et/ou H,
$X^5$       représente Mg, Ca, Sr et/ou Ba,
$X^6$       représente Si, Al, Ti et/ou Zr,
$X^7$       représente Ni et éventuellement un ou plusieurs éléments du groupe constitué de Cu, Zn, Co, Fe, Cd, Mn, Mg, Ca, Sr et Ba,
a        vaut 1 à 8,
b        vaut 0,2 à 5,
c        vaut 0 à 23,
d        vaut 0 à 50,
e        vaut 0 à 2,
f        vaut 0 à 5,
g        vaut 0 à 50,
h        vaut 0,1 à 50,
i        vaut 0 à 50,

x, y     représentent des nombres déterminés par la valence et la fréquence des éléments autres que l'oxygène dans (I)
p, q     représentent des nombres autres que 0 dont le rapport p/q va de 20:1 à 1:80,

qui contiennent la fraction $(A)_p$ sous la forme de zones A étendues en trois dimensions de composition chimique

A :

$$Mo_{12}V_aX^1_bX^2_cX^3_dX^4_eX^5_fX^6_gO_x$$

et la fraction $(B)_q$ sous la forme de zones A étendues en trois dimensions de composition chimique

B :

$$X^7_1Sb_hH_iO_y$$

les zones A, B étant distribuées les unes par rapport aux autres comme dans un mélange de A en fines particules et de B en fines particules,
à la condition que l'on utilise conjointement, pour la préparation des matières d'oxydes multimétalliques (I), au moins un oxométallate B préparé préalablement et séparément,

$$X^7_1Sb_hH_iO_y,$$

que l'on peut obtenir en préparant un mélange sec de sources appropriées des constituants élémentaires de l'oxométallate B, qui contiennent au moins une fraction de l'antimoine présentant un degré d'oxydation de +5, et en le calcinant à des températures allant de 200°C à < 600°C.

2.   Procédé de préparation d'oxydes multimétalliques selon la revendication 1, dans lequel on prépare préalablement un oxométallate B,

$$X^7_1Sb_hH_iO_y,$$

sous forme de fines particules puis on le transforme en un mélange sec avec des sources des constituants élémentaires des matières d'oxydes multimétalliques A

$$Mo_{12}V_aX^1{}_bX^2{}_cX^3{}_dX^4{}_eX^5{}_fX^6{}_gO_x,$$

dans le rapport quantitatif souhaité et qu'on calcine ce mélange à une température allant de 250°C à 500°C, **caractérisé en ce que** l'on peut obtenir au moins une fraction de l'oxométallate B en préparant un mélange sec de sources des constituants élémentaires de l'oxométallate B, qui contiennent au moins une fraction de l'antimoine présentant un degré d'oxydation de +5, et en le calcinant à des températures allant de 200°C à < 600°C.

3. Procédé de préparation oxydative par catalyse en phase gazeuse d'acide acrylique à partir d'acroléine, **caractérisé en ce que** l'on utilise un oxyde multimétallique selon la revendication 1 en tant que catalyseur.